# EUROPEAN PATENT SPECIFICATION

(11) **EP 0 151 579 B1**
(45) Date of publication and mention of the grant of the patent: **06.09.1995**
(21) Application number: 84901861.9
(22) Date of filing: 13.04.1984
(51) Int. Cl.: C12N 7/06

(54) **METHOD AND PRODUCTS FOR DETECTION OF HUMAN T CELL LEUKEMIA VIRUS**
VERFAHREN UND PRODUKTE ZUR FESTSTELLUNG VON MENSCHLICHEM T-ZELLEN-LEUKÄMIE-VIRUS
PROCEDE ET PRODUITS DE DETECTION DU VIRUS DE LA LEUCEMIE DE CELLULES T HUMAINES

(30) Priority: 27.04.1983 US 489187
(43) Date of publication of application: 21.08.1985
(73) Proprietor: THE PRESIDENT AND FELLOWS OF HARVARD COLLEGE, Cambridge, MA 02114 (US)
(72) Inventor: ESSEX, Myron, E., Sharon, MA 02356 (US); LEE, Tun-Hou, Newton, MA 02159 (US)
(74) Representative: Moon, Donald Keith
(86) International application number: US8400561
(87) International publication number: WO8404327

(56) References cited:
- Proceedings of the National Academy of Sciences, USA, Volume 77, pages 7415-7419, issued December 1980, POIESZ, B.J. et al, detection and isolation of Type C retrovirus particles from fresh and cultured lymphocytes of a patient with cutaneous T-Cell lymphoma.
- Journal of Virology, Volume 38, pages 906-915, issued June 1981, KALYANARAMAN, V.S. et al. immunological properties of a Type C retrovirus isolated from cultured human T-lymphoma cells and comparison to other mammalian retroviruses.
- Journal of Experimental Medicine, Volume 154, pages 333-346, issued August 1981, POSNER, L.E. et al, natural antibodies to the human T-cell lymphoma virusin in patients with cutaneous T cell lymphomas.
- Proceedings of the National Academy of Sciences, USA, Volume 78, pages 6476-6480, issued October 1981, HINUMA, Y. et al, adult T-cell leukemia: antigen in an ATL cell line and detection of antibodies to the antigen in human sera.
- Nature, Volume 294, pages 271-273, issued November 1981, KALYANARAMAN, V.S. et al, antibodies in human sera reactive against an internal structural protein of human T-cell lymphoma virus.
- Nature, Volume 294, pages 268-271, issued November 1981, POIESZ, B.J. et al, isolation of a new type C retrovirus (HTLV) in primary uncultured cells of a patient with sezary T-cell leukemia.
- Journal of Experimental Medicine, Volume 154, pages 1957-1964, issued December 1981, ROBERT-GUROFF, M. et al, detection of the human T-cell lymphoma virus p19 in cells of some patients with cutaneous T cell Lymphoma and leukemia using a monoclonal antibody.
- Science, Volume 215, pages 975-978, issued February 1982, ROBERT- GUROFF, M. et al, natural antibodies to human retrovirus HTLV in a cluster of japanese patients with adult T-cell leukemia.
- Proceedings of the National Academy of Sciences, USA, Volume 79, pages 1291-1294, issued February 1982, OROSZLAN, S. et al, primary structure analysis of the major internal protein p24 of human type C T-cell leukemia virus.
- Proceedings of the National Academy of Sciences, USA, Volume 79, pages 2031-2035, issued March 1982, YOSHIDA, M. et al, isolation and characteri-zation of retrovirus from cell lines of human adult T-cell leukemia and its Implication in the disease.
- Proceedings of the National Academy of Sciences, USA Volume 79, pages 1653-1657, issued March 1982, natural antibodies to the structural core protein p24 of human T-cell leukemia (lymphoma) retrovirus found in sera of leukemia patients in Japan.
- Journal of the National Cancer Institute, Volume 69, pages 981-985, issued October 1982, ESSEX, M., adult T-cell leukemia-lymphoma: role of a humanretrovirus.
- Virology, Volume 122, pages 297-305, issued October 1982, ROBERT- GUROFF, M. et al, identification of HTLV p19 specific natural human antibodies by competition with monoclonal antibody.
- Science Volume 218, pages 571-573, issued November 1982, KALYANARAMAN, V.S. et al, a new subtype of human T-cell leukemia virus (HTLV-II)associated with a T-cell variant of hairy cell leukemia.
- Science, Volume 220, pages 856-859, issued February 1983, POPOVIC, M. et al, isolation and transmission of human retrovirus (human T-cell leukemia virus).
- Science, Volume 220, pages 859-862, issued May 1983, ESSIX, M. et al, antibodies to cell membrane antigens associated with human T-cell leukemia virus in patients with aids.
- Science, Volume 220, pages 865-867, issued May 1983, GALLO, R.C. et al, isolation of human T-cell leukemia virus in acquired immune deficiency syndrome (aids).
- Science, Volume 220, pages 868-871, issued May 1983, BARRE-SINOUSS I,F. et al, isolation of a T-lymphotropic retrovirus from a patient at risk for acquired immune deficiency syndrome (aids).
- Proceedings of the National Academy of Science, USA, Volume 80, pages 3618-3622, issued June 1983, SEIKI, M. et al, human adult T-cell leukemia virus: complete nucleotide sequence of the retrovirus genome integrated in leukemia cell DNA.
- Science, Volume 223, pages 1083-1086, issued March 1984, MARX, P.A. et al, simian aids: isolation of a type D retrovirus and transmission of the disease.
- Journal of Experimental Medicine, Volume 159, pages 1117-1131, issued April 1984, PALKER, T.J. et al, monoclonal antibodies against human T cell leukemia-lymphoma virus (HTLV) p24 internal core protein.
- Yamamoto et al., Z. Naturforsch 37c, pp.731-732 (1982)
- Yamamoto et al., Int. J. Cancer 32, pp. 281-287 (1983)
- Hattori et al., Gann. 74, pp. 790-797 (1983)

## Description

This invention relates to a novel purified forms of glycoprotein found in the cell surface membrane of cells infected with human T cell leukemia virus, and to an assay for detecting in a biological specimen the presence of an antibody to the antigenic determinants present in said glycoproteins.

The human T cell leukemia virus (HTLV) is known to be closely associated with a particular type of human leukemia, the T cell type in adults. It has also been shown that all people whose bodies contain antibodies to this virus are apparently latently infected with the virus. Essex, J.N.C.I., Vol. 69, 981-5 (1982). The major core proteins of the virus have been studied and an immunofluorescent assay procedure for antibodies to the infected cells has been described which involves fixing cells from a cell line of infected human cells such as MT1 or MT2, contacting the fixed cells with the test serum to be assayed, and determining whether bonding of the serum to the cell surface has occurred by subsequently contacting with a fluorescent-labelled antibody to human IgG. Hinuma et al., Proc.Natl.Acad.Sci., Vol. 78, 6476-6480 (1981). This assay is tedious and difficult to use because only 1 to 5% of the fixed cells display the necessary antigenic characteristics. It has also been proposed to employ a somewhat analogous cell surface immunofluorescent assay in which a culture of infected cells is incubated with test serum, then with fluorescent-labelled rabbit antibody to human IgG to determine whether bonding of serum to cells has occurred. Robert-Guroff et al., Science, Vol. 215, 975-978 (1982). A radioimmunoassay for antibodies to p24 and p19, two of the core proteins of HTLV, has also been described. Posner et al., J.Exp.Med., Vol. 154, 333-344 (1981). However, this assay fails to produce positive results for all individuals who have been exposed to the infecting virus and are therefore at risk, but who do not have detectable levels of antibodies to these two antigens.

### Summary of the Invention

It has now been found that particular polypeptides or glycoproteins present on the cell surface of human T cells infected with HTLV when purified and isolated contain an antigenic determinant or determinants which provide a high degree of sensitivity and immunospecificity for antibody to human cells infected with HTLV, to human T cell leukemia cells, and/or to HTLV. Consequently, the substantially pure glycoproteins or their unglycosylated moleties are useful as a diagnostic tool for assaying biological specimens to determine whether they contain cells which have been infected by HTLV. Other polypeptides containing immunologically cross-reactive antigenic determinants are useful for the same purpose. By "polypeptides containing immunologically cross-reactive antigenic determinants" is meant polypeptides having in common antigenic determinants with which a given antibody will react. Such other polypeptides include the unglycosylated moieties of the glycoproteins. Other useful polypeptides or proteins, which have the necessary immunogenic determinants, include synthetic polypeptides. They also include antibodies or fragments thereof which are anti-idiotypic towards the active determinant or determinants on the glycoprotein of the invention. It has recently been shown that anti-idiotypic monoclonal antibodies can induce an immune response against, and protect against infection by infectious organisms carrying antigenic determinants which are the same or substantially the same as those on the anti-idiotypic antibodies (Fields et al., Nature, 300:19-23 (1982); Sacks et al., J.Exp.Med. 4:1108-1119 (1982)). It has also been shown that anti-idiotypic reagents are useful as diagnostic tools for the detection of antigens carrying sites which are immunologically cross-reactive with those on the antibodies (Potocnjak et al., Science 215: 1637-1639 (1982) herein incorporated by reference). Thus, an assay for HTLV could be carried out with the aid of a anti-idiotypic antibody or immunologically active fragment thereof which carries an antigenic site or sites thereon which are immunologically similar to the antigenic site or sites on the glycoprotein of the invention. Such anti-idiotypic antibodies can be raised against first antibodies having specificity against the antigenic sites on the glycoprotein of the invention (i.e. the anti-idiotypic antibodies are anti-antibodies). Preferably monoclonal anti-idiotypic antibodies are used.

An assay for HTLV infection is important because the virus can be readily transferred from the peripheral blood leukocytes of antibody-positive people to leukocytes of antibody-negative people when the two are cultivated together. Popovic et al., Science, Vol. 219, 856-859 (1983). Consequently, it appears that there is great risk of infection involved in whole blood transfusions when the transfused blood contains infected cells. In addition, the assay is of importance because biological specimens from individuals exhibiting acquired immunodeficient syndrome (AIDS) also give a positive test for antibodies to the antigenic determinant of the novel glycoprotein, thus facilitating diagnosis of that disease.

Consequently, the invention also embraces the method of assaying a biological specimen for the presence of antibody to HTLV-infected cells which comprises incubating said specimen with a polypeptide having an antigenic determinant or determinants immunologically cross-reactive with those of a glycoprotein having a molecular weight of approximately 61,000-68,000 daltons, of which approximately 46-48,000 daltons is the unglycosylated moiety, which glycoprotein occurs on the cell surface of cells infected with HTLV, and determining whether or not an immunocomplex is formed between said antibody and said polypeptide.

The invention also embraces a method of assaying a biological specimen for the presence of antigenic determinant or determinants immunologically cross-reactive with the determinants of the glycoprotein of molecular weight 61,000-68,000 daltons. The determinants to be may occur on the stated glycoprotein itself or on other polypeptides. They may be in free circulation in the body fluids or in lymphocytes. The assay can be carried out by known immunoassay methods, using antibodies, monoclonal or polyvalent, having immune reactivity with the antigenic determinants found on the stated glycoproteins. For example competitive immunoassays or immunometric (sandwich) assays can be used.

The glycoprotein of the present invention has a molecular weight of approximately 61,000-68,000 daltons as determined by sodium dodecyl sulfate (SDS) gel electrophoresis and is soluble in SDS buffer consisting of 0.15 M sodium chloride, 0.05 M Tris hydrochloride pH 7.2, 1% Triton X-100, 1% sodium deoxycholate, 0.1% sodium dodecylsulfate, and 1 mM phenylmethylsulfonyl fluoride. Triton X-100 is a nonionic detergent (octylphenoxy polyethoxy (9-10) ethanol). The unglycosylated moiety of the 61,000-68,000 d glycoprotein has a molecular weight of approximately 46,000-48,000 daltons and contains substantially the same antigenic determinant or determinants as does the glycoprotein itself.

The glycoprotein can be obtained from HTLV-infected cells. A variety of cell lines have been prepared, which are permanently and persistently infected with HTLV; among them can be mentioned MJ, C5-MJ, C91PL, and HUT-102. It may be that the exact sizes of the novel glycoprotein is slightly different in different lines; however, the common immunologically cross-reactive portion of the glycoprotein is the same regardless of cell line, since it is a protein induced by HTLV. Thus, any cell which harbors the virus may be an appropriate source for the novel glycoprotein. In order to obtain the protein from any infected cells carrying the virus, the cells are metabolically labelled (e.g. with ³⁵S-methionine) and immunoprecipitated with antisera obtained from HTLV infected subjects. The novel glycoprotein can then be detected and isolated by gel electrophoresis. By "HTLV" as used in the present specification and claims it is meant to include the virus generically. Thus any and all forms, subtypes, or variations of the virus are included.

For example, the glycoprotein is present at the cell surfaces of the human T cell leukemia cell cultures MJ, C5-MJ, C91PL and HUT-102. A specimen of MJ and of C5-MJ have been deposited with the American Type Culture Collection on April 26, 1983 as ATCC Nos. CRL-8294 and CRL-8293, respectively. The glycoprotein can readily be separated from the cells of these cell lines by lysis thereof and SDS gel electrophoresis.

The purified and isolated glycoprotein or any antigen immunologically cross-reactive therewith can be employed as a standard antigen in any conventional assay procedure for detection in biological specimens of the presence of antibodies specific thereto, hence of the presence in the specimen of cells infected with HTLV and/or symptomatic of AIDS. The antibodies specific to such HTLV antigens are not found in patients suffering from diseases such as hepatitis which are not accompanied by HTLV infection.

The glycoprotein or polypeptides immunologically cross-reactive therewith can be labelled by conventional procedures with ¹²⁵I or ³⁵S or ³H for use in radioimmunoassay, with fluorescein for fluorescent immunoassay, with enzyme for enzyme immunoassay or with biotin, for biotin-avidin linked assays. It can be employed, labelled or unlabelled as desired, in competitive immunoassays, as well as in double antibody assays using two antibodies, either of the idiotype:anti-idiotype variety or more particularly of the second antibody type using an anti-Fc antibody, or other assays.

Alternatively, the novel glycoprotein or polypeptides immunologically cross-reactive therewith could be immobilized on an insoluble phase, such as an insoluble resin, and detection of the anti-glycoprotein antibodies is carried out by measuring their binding to the insoluble phase. Insoluble phases also include latex particles, which, when coated with the novel glycoprotein or its immunologically cross-reactive polypeptides and subjected to anti-glycoprotein antibody, will agglutinate. Yet other insoluble phases include test tubes, vials, titration wells, and the like, to which the novel glycoprotein or its immunologically cross-reactive polypeptide can be bound, and antibody thereto detected by double antibody techniques or Protein-A dependent techniques.

The assay for antibody to HTLV may utilize the glycoprotein or glycoproteins of m.w. 61-68,000, in crude form, and is not limited to using the protein in substantially pure form. In one embodiment, the assay for the presence of antibodies against HTLV may include detection of additional antibodies in the specimen such as those against HTLV core proteins p19, p24 or a mixture of both of the latter. In this embodiment the method comprises incubating the specimen with a reagent comprising 1) glycoprotein of the invention (i.e. 61,000-68,000 or immunocross-reactive polypeptides) and, optionally, 2) HTLV core proteins p24 or p19 or both, and determining whether or not an immunocomplex is formed between antibodies in the specimen and the reagent.

The elements necessary for carrying out the diagnostic methodology described hereinbefore may be present in a kit. Such kit comprises a carrier being compartmentalized to receive therein one or more containers, each of said containers comprising one or more elements necessary to carry out the tests.

For example, the first container may contain the purified glycoprotein or its immunologically cross-reactive polypeptides in detectably labelled or in insolubilized form.

A second container may comprise anti IgG antibody, polyclonal or monoclonal, useful in double antibody binding assay, or elements needed for detection of the label on the glycoprotein or its immunologically cross-reactive polypeptides (e.g. chromogenic substrates).

Additional containers may comprise varying amounts of glycoprotein or its immunologically cross-reactive polypeptides which can be used to prepare a standard curve into which experimental results can be interpolated. The materials may be present in the kit by themselves, in solution, freeze-dried, or in admixture with other inert materials, such as inert proteins, and the like.

The biological specimens tested may include blood, serum, lymphocytes, urine, tissues, saliva, feces, and the like. Of particular interest is the screening of blood in blood banks, to assure that the blood is not contaminated with HTLV. Screening of blood-derived products, such as vaccines, can also be done by the methods of the invention.

The following specific examples are intended to illustrate more fully the nature of the invention without acting as a limitation upon its scope.

### Example 1

### Preparation of Labelled Glycoprotein

A. Human T cell leukemia cells from the two cell lines MJ and C5-MJ were separately harvested at their log phase of growth. After one wash with methionine-free McCoy's 5A medium, each sample of the cells was resuspended in a labelling medium consisting of methionine-free McCoy's 5A, 10% phosphate-buffered saline (PBS), dialyzed fetal bovine serum, and 100 »Ci/ml of ³⁵S-methionine. At the end of a 2 to 4 hour pulsing, the radioactive labelled cells were washed three times with cold PBS. The cell pellet was then lysed with 0.6 ml to 1.0 ml of cold lysis buffer (RIPA) (0.15 M sodium chloride, 0.05 M Tris-hydrochloride pH 7.2, 1% Triton X-100 wetting agent, 1% sodium deoxycholate, 0.1% sodium dodecyl sulfate, and 1 mM phenylmethylsulfonyl fluoride). After 10 minutes of intermittent vortexing, the mix was centrifuged for 1 hour at 100,000 xg at 4°C. The lysate supernatant was precleared of nonspecific binding components by absorption for one hour at 4°C. on carbohydrate beads (Sepharose CL-4B) coated with protein-A.
B. Labelling of cell surface membrane protein was carried out by lactoperoxidase catalyzed radioiodination. Three aliquots of 5 x 10⁶ cells of each line with greater than 99% viability were iodinated separately with 1 mCi of carrier-free sodium ¹²⁵iodide in the presence of 50 »l carrier-supported lactoperoxidase and glucosidase (Enzymobeads, Bio-Rad Labs.) and 25 »l of 1% beta-D-glucose. After the reaction was terminated, three aliquots of iodinated cells were pooled and subjected to the same lysing and preclearing procedures as described in (A) above.
C. MJ and C5-MJ cells at their peak log phase of growth were separately harvested, then resuspended in glucose-free RPMI-1640 medium supplemented with 1 mg/ml of sodium pyruvate for 2 hours. After this glucose starvation, the cells were labelled with 100 »Ci/ml of ³H-glucosamine (New England Nuclear) for 5-6 hours. The procedure results in tritium labelling of only the glycoproteins present in the cells. The labelled cells were then subjected to lysing and preclearing procedures as described in (A) above.

### Preparation of Labelled Unglycosylated Moiety of Glycoprotein

D. MJ and C5-MJ cells at their peak log phase of growth were separately harvested and resuspended in McCoy's 5A medium supplemented with 10% fetal bovine serum, 1% of antibiotic-antimycotic mixture, and 20 »g/ml of tunicamycin for 2 hours. After this trimming step, the cells were labelled with 100 »Ci/ml of ³⁵S-methionine in the presence of 20 »g/ml of tunicamycin for 2 hours. The labelled material was then subjected to the same lysing and preclearing procedures as described in (A) above.

### Formation of Protein-Antibody Complex

E. There were bound to aliquots of protein-A-coated beads (a) positive reference blood serum for individual known to harbor antibodies against cells infected with HTLV; (b) negative control serum from individuals free from infection; and (c) serum from unknown individuals to be tested. Each aliquot of coated beads was then reacted with an aliquot of each precleared lysate obtained from MJ and from C5-MJ from paragraph (A) above at 4°C. for 1-2 hours to permit complex formation or immunoprecipitation to occur between the bonded lysate protein and any antibody present in the sera. At the end of the reaction the beads were washed 4 times with the buffer (RIPA) and once with a buffer containing 0.05 M Tris-hydrochloride pH 7.2 and 0.15 M sodium chloride to remove uncomplexed lysate protein.

The beads were then immersed in a sample buffer (0.1 M Cleland's reagent, 2% sodium dodecylsulfate, 0.08 M Tris-hydrochloride pH 6.8, 10% glycerol, and 0.2% Bromphenol Blue) and subjected to boiling at 100°C. for 2 minutes to elute proteins from the beads and to dissociate complexes.

### Characterization of Protein

F. Each sample from the foregoing procedure (A) was analyzed by electrophoresis, the proteins being separated on a 12.5% SDS-polyacrylamide gel with 3.5% stacking gel using the Laemmli buffer system. Molecular weight markers were run simultaneously in a parallel column. The markers used included ¹⁴C-labelled phosphorylase b (92,500), bovine serum albumin (68,000), and ovalbumin (46,000) carbonic anhydrase (30,000) and cytochrome C (12,000). For visualization by fluorography, the gels were first fixed with 10% acetic acid, 10% trichloroacetic acid, and 30% methanol for 1 hour, then immersed in Enhancer solution for 1 hour. After rinsing with distilled water and drying under vacuum, the gels were exposed on Kodak SB-5 film to provide autoradiographs.

The spots of purified and isolated 61,000-68,000 molecular weight glycoprotein appeared prominently in all of the columns of serum (a), but in none of those of serum (b). In contrast, the core proteins p19 and p24 failed to appear in all samples of serum (a). Appearance of the glycoprotein in the unknown sera (c) indicated the presence in the sera of cells infected with HTLV. Sera from individuals exhibiting AIDS also exhibited prominent evidence of antibodies to the 61,000-68,000 MW glycoprotein in this procedure.

Similar results have been obtained by substituting for the labelled glycoproteins of paragraph A, either of those of paragraphs B or C. There could also be substituted for it the labelled unglycosylated moiety of paragraph D, in which case the purified and isolated labelled moiety appears in the gel column at a location corresponding to molecular weight 46,000-48,000. Unlabelled glycoprotein and unlabelled unglycosylated moiety can be obtained by omitting the labelling steps in the foregoing procedures.

Similar results have been obtained by substituting cell lines C91PL or HUT-102 in place of MJ or C5MJ.

Amino Acid Sequence Analysis. [³⁵S]cysteine-labeled glycoproteins having a molecular weight of approximately 61,000 daltons, were precipitated from 36 x 10⁶ Hut 102 cells metabolically labeled with 5mCi of [³⁵S]cysteine (specific activity 1011.2Ci/mmol, New England Nuclear) in 30 ml of cysteine-free RPMI-1640 media containing 15% fetal bovine serum (Grand Island Biological Co.) for 10 hours. The glycoprotein was excised from NaDodSO₄ polyacrylamide gels and then subjected to electrophoretic elution in the presence of 50mM Tris-acetate pH 7.8 buffer containing 0.01% NaDodSO₄ for 12-16 hours. Samples were dialyzed once with 10mM of ammonium bicarbonate buffer containing 0.002% NaDodSO₄ for 12-16 hours. One more dialysis was done with 10mM ammonium bicarbonate buffer without NaDodSO₄ for 12-16 hours. Each sample was then lyophilized and amino acid sequence analysis was conducted by the procedures described by Coligan et al., J. Immun. Meth., Vol. 47, 1-11 (1981) and Meth. Enzymol., Vol. 91, 413-434 (1983). Briefly, automated Edman degradation of radiolabeled peptides was performed utilizing a Beckman 890C sequencer with cold trap modification and 0.1 M Quadrol program 121078. The butyl chloride extract from each sequence step was dried using N₂ evaporation in 7.0 ml scintillation vials. After addition of Biofluor (NEN), radioactivity was determined on a Beckman LS9080 liquid scintillation counter. [³⁵S] peaks were found at residues 6, 7, 21 and 28, indicating the presence of cysteine at these positions in the protein sequence of the 61,000-68,000 d glycoprotein. The repetitive yield for this sequence was 88%, indicating that all the residues are in the same sequence. Small peaks at positions 4 and 13 are presumably from small amounts (less than 5%) of contaminating proteins. These results indicate that the 61,000-68,000 mw glycoprotein is encoded, at least in part, by the env gene of HTLV and that the leader sequence of the env gene consists of 20 residues.

Similar analysis was carried out with [³⁵S] cysteine labeled glycoprotein having molecular weight of approximately 67,000 d. Cysteine residues were detected at positions 6, 7 and 21 when the first 22 NH₂-terminal residues were analyzed. This shows that this glycoprotein is also encoded, at least in part, by the same NH₂-terminal end of the env gene.

## Claims

1. A substantially pure polypeptide having an antigenic determinant or determinants immunologically cross-reactive with the determinants of a glycoprotein having a molecular weight determined by SDS polyacrylamide gel electrophoresis of approximately 61,000-68,000 daltons, of which approximately 46-48,000 daltons is the molecular weight of the unglycosylated moiety, said glycoprotein being present on the cell surface of cells infected with human T-cell leukemia virus.

2. The polypeptide of claim 1 which is the HTLV-associated cell surface glycoprotein of claim 1.

3. A polypeptide as claimed in claim 1 which comprises the unglycosylated moiety of the HTLV-associated cell surface glycoprotein of claim 1.

4. A polypeptide as claimed in claim 1 in which said glycoprotein is present on the cell surface of human T-cell leukemia cell lines CRL-8294 or CRL-8293.

5. A polypeptide as claimed in claims 2 or 3 which is obtained from MJ, C5-MJ, C91PL or HUT-102 cells.

6. A polypeptide as claimed in claim 1 which comprises an anti-idiotypic antibody having antigenic determinants which are immunologically cross-reactive with those of said glycoprotein.

7. A polypeptide as claimed in any of claims 1, 2, 3, 4 or 6 which is detectably labelled.

8. The polypeptide of claim 7 wherein said label is selected from the group consisting of a radiolabel, a fluorogenic label, an enzyme label or a biotin label.

9. The polypeptide of any of claims 1, 2, 3 or 4 which is bound to an insoluble phase.

10. The polypeptide of claim 9 wherein said solid phase is a latex particle or an insoluble resin.

11. A process for preparing the glycoprotein of claim 2 which comprises culturing MJ or C5-MJ cells under appropriate culturing conditions, and
extracting said glycoprotein from said cells.

12. The substantially unglycosylated moiety of a glycoprotein having a molecular weight determined by SDS polyacrylamide gel electrophoresis of approximately 61,000-68,000 daltons of which approximately 46,000-48,000 daltons is the unglycosylated moiety, which glycoprotein occurs on the cell surface of cells infected with HTLV.

13. The moiety as claimed in claim 12 which is detectably labelled.

14. The moiety as claimed in claim 12 which is bound to an insoluble phase.

15. The method of assaying a biological specimen for the presence of antibody to HTLV-infected cells which comprises
incubating said specimen with a polypeptide having an antigenic determinant or determinants immunologically cross-reactive with the determinants of a glycoprotein having a molecular weight determined by SDS polyacrylamide gel electrophoresis of approximately 61,000-68,000 daltons, of which approximately 46-48,000 daltons is the molecular weight of the unglycosylated moiety, said glycoprotein being present on the cell surface of cells infected with human T-cell leukemia virus, and claimed in claim 1, and
determining whether or not an immunocomplex is formed between said antibody and said polypeptide.

16. The method as claimed in claim 15 in which said polypeptide is said glycoprotein having a molecular weight determined by SDS polyacrylamide gel electrophoresis of approximately 61,000-68,000 daltons.

17. The method as claimed in claim 15 in which said polypeptide is said unglycosylated moiety having a molecular weight determined by SDS polyacrylamide gel electrophoresis of approximately 46,000-48,000 daltons.

18. The method as claimed in any of claims 15-18 including the step of labelling said polypeptide.

19. The method as claimed in any of claims 15-18 which is an enzyme linked immunoassay.

20. The method as claimed in any of claims 15-18 which is a radioimmunoassay.

21. The method as claimed in any of claims 15-18 which is a latex particle agglutination assay.

22. The method as claimed in any of claims 15-18 which is a fluorescence assay.

23. The method as claimed in any of claims 15-18 which is a double antibody immunoassay.

24. A method of detecting the presence in a biological specimen of an antigenic determinant or determinants immunologically cross-reactive with those of the polypeptide claimed in claim 1 which comprises:
incubating said specimen with antibodies against the polypeptide of claim 1, and
determining whether or not an immunocomplex is formed between said antibodies and said polypeptides.

25. The method as claimed in claim 24 wherein said specimen comprises human lymphocytes.

26. The method as claimed in claim 24 wherein said antibodies are against said glycoprotein having a molecular weight determined by SDS polyacrylamide gel electrophoresis of 61,000-68,000 daltons.

27. The method as claimed in claim 24 wherein said antibodies are against the unglycosylated moiety of said glycoprotein having a molecular weight determined by SDS polyacrylamide gel electrophoresis of 61,00-68,000 daltons.

28. The method as claimed in claim 24 wherein said antibodies are against a glycoprotein which occurs on the cell surface of MJ or C5-MJ cells.

29. A kit useful for assaying a sample for the presence of antibody to HTLV-infected cells, said kit being compartmentalized to receive in close confinement therein one or more containers which comprises
a first container containing the polypeptide of any of claims 1, 2, 3, 4 or 6
a second container containing means for detecting the formation of an immunocomplex between said antibody and said polypeptide.

## Patentansprüche

1. Im wesentlichen reines Polypeptid mit einer antigenen Determinante oder mit Determinanten, die mit Determinanten eines Glykoproteins immunologisch kreuzreagiert bzw. kreuzreagieren, das bestimmt mittels SDS-Polyacrylamid-Gelelektrophorese ein Molekulargewicht zwischen 61 000 und 68 000 Dalton hat, wovon näherungsweise 46 000 bis 48 000 Dalton des Molekulargewichts auf die nicht glykolysierte Komponente entfallen und wobei das Glykoprotein auf der Zelloberfläche von Zellen auftritt, die mit menschlichem T-Zellen-Leukämie-Virus infiziert sind.

2. Polypeptid nach Anspruch 1, das das HTLV-assoziierte Zelloberflächenglykoprotein nach Anspruch 1 ist.

3. Polypeptid nach Anspruch 1, das eine nicht glykolysierte Komponente des HTLV-assoziierten Zelloberflächenglykoproteins nach Anspruch 1 aufweist.

4. Polypeptid nach Anspruch 1, bei dem das Glykoprotein auf der Zelloberfläche von menschlichen T-Zell-Leukämiezellen der Linie CRL-8294 oder CRL-8293 vorhanden ist.

5. Polypeptid nach den Ansprüchen 2 oder 3, das aus MJ, C5-MJ, C91PL oder HUT-102 Zellen gewonnen ist.

6. Polypeptid nach Anspruch 1, das einen anti-idiotypischen Antikörper mit antigenen Determinanten aufweist, die mit denen des Glykoproteins immunologisch kreuzreagiert.

7. Polypeptid nach einem der Ansprüche 1, 2, 3, 4 oder 6, das erkennbar markiert ist.

8. Polypeptid nach Anspruch 7, wobei die Markierung aus einer Gruppe ausgewählt ist, zu der eine radioaktive, eine fluorogene, eine enzymatische oder eine Biotinmarkierung gehört.

9. Polypeptid nach einem der Ansprüche 1, 2, 3 oder 4, das mit einer unlöslichen Phase gekoppelt ist.

10. Polypeptid nach Anspruch 9, wobei die feste Phase ein Latexpartikel oder ein unlösliches Herz ist.

11. Verfahren zur Herstellung des Glykoproteins nach Anspruch 2, das es beinhaltet, MJ oder C5-MJ-Zellen unter geeigneten Kulturbedingungen zu kultivieren und
das Glykoprotein aus den Zellen zu extrahieren.

12. Im wesentlichen nicht glykolysierte Komponente eines Glykoproteins mit einem mittels SDS-Polyamid-Gelelektrophorese bestimmten Molekulargewicht von näherungsweise zwischen 61 000 und 68 000 Dalton, wovon näherungsweise 46 000 bis 48 000 Dalton auf die nicht glykolysierte Komponente entfallen und wobei das Glykoprotein auf der Zelloberfläche von Zellen auftritt, die mit HTLV infiziert sind.

13. Komponente nach Anspruch 12, die erkennbar markiert ist.

14. Komponente nach Anspruch 12, die an eine unlösliche Phase gekoppelt ist.

15. Verfahren zum Testen von biologischen Proben auf das Vorhandensein von Antikörpern gegen HTLV-infizierte Zellen, gemäß dem:
die Proben mit einem Polypeptid inkubiert werden, das eine antigene Determinante oder Determinanten aufweist, die mit den Determinanten eines Glykoproteins immunologisch kreuzreagiert bzw. kreuzreagieren, das ein mittels SDS-Polyacryl-Gelelektrophorese bestimmtes Molekulargewicht von näherungsweise 61 000 bis 68 000 Dalton aufweist, wovon näherungsweise 46 000 bis 48 000 Dalton auf das Molekulargewicht der unglykolysierten Komponente entfallen und wobei das Glykoprotein auf der Zelloberfläche von Zellen auftritt, die mit menschlichem T-Zell-Leukämie-Virus (HTLV) infiziert sind, nach Anspruch 1 und
festgestellt wird, ob zwischen dem Antikörper und dem Polypeptid ein Immunkomplex gebildet wird oder nicht.

16. Verfahren nach Anspruch 15, bei dem das Polypeptid das Glykoprotein mit einem mittels SDS-Polyacryl-Gelelektrophorese bestimmten Molekulargewicht von näherungsweise 61 000 bis 68 000 Dalton ist.

17. Verfahren nach Anspruch 15, bei dem das Polypeptid die unglykolysierte Komponente mit einem mittels SDS-Polyacryl-Gelelektrophorese bestimmten Molekulargewicht von näherungsweise 46 000 bis 48 000 Dalton ist.

18. Verfahren nach einem der Ansprüche 15 bis 18, das den Schritt der Markierung des Polypeptids umfaßt.

19. Verfahren nach einem der Ansprüche 15 bis 18, das ein enzymgekoppelter Immuntest ist.

20. Verfahren nach einem der Ansprüche 15 bis 18, das ein Radioimmuntest ist.

21. Verfahren nach einem der Ansprüche 15 bis 18, das ein Test mit Agglutinierung von Latexpartikeln ist.

22. Verfahren nach einem der Ansprüche 15 bis 18, das ein Fluoreszenztest ist.

23. Verfahren nach einem der Ansprüche 15 bis 18, das ein Doppelantikörperimmunotest ist.

24. Verfahren, um festzustellen, ob in einer biologischen Probe eine antigene Determinante oder Determinanten vorhanden sind, die mit denen des Polypeptids nach Anspruch 1 immunologisch kreuzreagieren, wobei gemäß dem Verfahren:
die Probe mit Antikörpern gegen das Polypeptid nach Anspruch 1 inkubiert wird und
festgestellt wird, ob zwischen den Antikörpern und den Polypeptiden Immunokomlexe gebildet werden oder nicht.

25. Verfahren nach Anspruch 24, wobei die Proben menschliche Lymphozyten enthalten.

26. Verfahren nach Anspruch 24, bei dem die Antikörper gegen das Glykoprotein gerichtet sind, das ein mittels SDS-Polyacryl-Gelelektrophorese bestimmtes Molekulargewicht von zwischen 61 000 und 68 000 Dalton aufweist.

27. Verfahren nach Anspruch 24, bei dem die Antikörper gegen die unglykolisierte Komponente des Glykoproteins mit einem mittels SDS-Polyacryl-Gelelektrophorese bestimmten Molekulargewicht von 61 000 bis 68 000 Dalton gerichtet sind.

28. Verfahren nach Anspruch 24, bei dem die Antikörper gegen ein Glykoprotein gerichtet sind, das auf der Zelloberfläche von MJ oder C5-MJ-Zellen auftritt.

29. Zusammenstellung zur Verwendung zum Testen einer Probe auf das Vorliegen von Antikörpern gegen HTLV-infizierte Zellen, wobei die Zusammenstellung in Abteile aufgeteilt ist, um in geschlossenen Abteilungen einen oder mehrere Behälter aufzunehmen, zu denen gehört:
ein erster Behälter, der das Polypeptid nach einem der Ansprüche 1, 2, 3, 4 oder 6 enthält,
ein zweiter Behälter, der Mittel enthält, um die Bildung eines Immunokomplexes zwischen dem Antikörper und dem Polypeptid zu erkennen.

## Revendications

1. Polypeptide pratiquement pur, possédant un ou des déterminants antigéniques donnant une réaction croisée d'un point de vue immunologique avec les déterminants d'une glycoprotéine ayant un poids moléculaire d'environ 61000 à 68000 daltons déterminé par électrophorèse sur gel de polyacrylamide SDS, dont le reste non glycosylé a un poids moléculaire d'approximativement 46000 à 48000 daltons, ladite glycoprotéine étant présente à la surface des cellules infectées par le virus de la leucémie des cellules T humaines.

2. Polypeptide, selon la revendication 1, qui est la glycoprotéine présente à la surface d'une cellule associée au virus HTLV de la revendication 1.

3. Polypeptide, selon la revendication 1, comprenant le reste non glycosylé de la glycoprotéine présente à la surface d'une cellule associée au virus HTLV de la revendication 1.

4. Polypeptide, selon la revendication 1, dans lequel ladite glycoprotéine est présente à la surface d'une cellule provenant des lignées cellulaires de la leucémie de cellules T humaines CRL-8294 ou CRL-8293.

5. Polypeptide, selon l'une quelconque des revendications 2 ou 3, obtenu à partir de cellules MJ, C5-MJ, C91PL ou HUT-102.

6. Polypeptide, selon la revendication 1, comprenant un anticorps anti-idiotypique ayant des déterminants antigéniques qui réagissent de manière croisée d'un point de vue immunologique avec ceux de ladite glycoprotéine.

7. Polypeptide, selon l'une quelconque des revendications 1, 2, 3, 4 ou 6, qui est marqué de façon détectable.

8. Polypeptide selon la revendication 7, marqué à l'aide d'un agent choisi dans le groupe constitué par un marqueur radioactif, un marqueur fluorogénique, un marqueur enzymatique ou par de la biotine.

9. Polypeptide selon l'une quelconque des revendications 1,2,3 ou 4, qui est lié à une phase solide insoluble.

10. Polypeptide selon la revendication 9, dans lequel ladite phase solide est une particule de latex ou une résine insoluble.

11. Procédé de préparation de la glycoprotéine de la revendication 2 comprenant l'étape de culture des cellules MJ ou C5-MJ dans des conditions de cultures appropriées et l'étape d'extraction de ladite glycoprotéine à partir desdites cellules.

12. Reste pratiquement non glycosylé d'une glycoprotéine ayant un poids moléculaire d'environ 61000 à 68000 daltons déterminé par électrophorèse sur gel de polyacrylamide SDS, dont le reste non glycosylé a un poids moléculaire d'approximativement 46000 à 48000 daltons, ladite glycoprotéine étant présente à la surface des cellules infectées par le virus HTLV.

13. Reste selon la revendication 12 qui est marqué de façon détectable.

14. Reste selon la revendication 12 qui est fixé à une phase insoluble.

15. Méthode de détection dans un échantillon biologique de la présence d'anticorps dirigés contre des cellules infectées par HTLV dans laquelle on met en contact ledit échantillon avec un polypeptide selon la revendication 1, possédant un ou des déterminants antigéniques donnant une réaction croisée d'un point de vue immunologique avec les déterminants d'une glycoprotéine ayant un poids moléculaire d'environ 61000 à 68000 daltons déterminé par électrophorèse sur gel de polyacrylamide SDS, dont le reste non glycosylé a un poids moléculaire d'approximativement 46000 à 48000 daltons, ladite glycoprotéine étant présente à la surface des cellules infectées par le virus de la leucémie de cellules T humaines et dans laquelle on détermine si un immunocomplexe se forme entre ledit anticorps et ledit polypeptide.

16. Méthode selon la revendication 15 dans laquelle ledit polypeptide est représenté par ladite glycoprotéine ayant un poids moléculaire d'environ 61000 à 68000 daltons déterminé par électrophorèse sur gel de polyacrylamide SDS.

17. Méthode selon la revendication 15 dans laquelle ledit polypeptide est représenté par ledit reste non glycosylé ayant un poids moléculaire d'environ 46000 à 48000 daltons déterminé par électrophorèse sur gel de polyacrylamide SDS.

18. Méthode selon l'une quelconque des revendications 15 à 18 comprenant l'étape de marquage dudit polypeptide.

19. Méthode selon l'une quelconque des revendications 15 à 18 qui est un immunoessai à enzyme fixée.

20. Méthode selon l'une quelconque des revendications 15 à 18 qui est un radioimmunoessai.

21. Méthode selon l'une quelconque des revendications 15 à 18 qui est un test d'agglutination sur particule de latex.

22. Méthode selon l'une quelconque des revendications 15 à 18 qui est un test par fluorescence.

23. Méthode selon l'une quelconque des revendications 15 à 18 qui est un immunoessai à double anticorps.

24. Méthode de détection de la présence dans un échantillon biologique d'un déterminant ou de déterminants réagissant de manière croisée d'un point de vue immunologique avec les déterminants du polypeptide selon la revendication 1 comprenant l'étape de mise en contact dudit échantillon avec lesdits anticorps dirigés contre le polypeptide selon la revendication 1 et l'étape de détermination de la formation ou non d un immunocomplexe entre lesdits anticorps et ledit polypeptide.

25. Méthode selon la revendication 24 dans laquelle ledit échantillon comprend des lymphocytes humains.

26. Méthode selon la revendication 24 dans laquelle lesdits anticorps sont dirigés contre ladite protéine ayant un poids moléculaire de 61000 à 68000 daltons déterminé par électrophorèse sur gel de polyacrylamide SDS.

27. Méthode selon la revendication 24 dans laquelle lesdits anticorps sont dirigés contre le reste non glycosylé de ladite protéine ayant un poids moléculaire de 61000 à 68000 daltons déterminé par électrophorèse sur gel de polyacrylamide SDS.

28. Méthode selon la revendication 24 dans laquelle lesdits anticorps sont dirigés contre une glycoprotéine qui se trouve à la surface cellulaire des cellules MJ ou C5-MJ.

29. Coffret de diagnostic pour la détection dans un échantillon de la présence d'un anticorps dirigé contre des cellules infectées par HTLV, ledit coffret étant compartimenté de façon à y admettre en étroit confinement un ou plusieurs récipients et qui comprend un premier récipient contenant le polypeptide selon l'une quelconque des revendications 1, 2, 3, 4 ou 6 et un second récipient contenant des moyens de détection de la formation d'un immunocomplexe entre ledit anticorps et ledit polypeptide.
